# EUROPEAN PATENT SPECIFICATION

(11) **EP 1 329 206 B1**
(45) Date of publication and mention of the grant of the patent: **05.07.2006**
(21) Application number: 02011395.7
(22) Date of filing: 24.05.2002
(51) Int. Cl.: G02C 7/04, A61F 2/16, A61F 9/013

(54) **Eye lens profile capable of correcting ametropias associated with astigmatism**
Linsenprofil für Augen zum Korrigieren von Fehlsichtigkeiten in Verbindung mit Astigmatismus
Profilé de lentille pour des Yeux pour la correction des ametrophies associés à l'astigmatisme

(30) Priority: 21.12.2001 IT MI20012765
(43) Date of publication of application: 23.07.2003
(73) Proprietor: Euro Lens Technology S.p.A, 24079 Staranzano (GO) (IT)
(72) Inventor: Altieri, Andrea, 34074 Monfalcone (GO) (IT)
(74) Representative: Ferraiolo, Ruggero

(56) References cited:
- EP-A- 0 996 024
- US-B1- 6 179 420
- US-B1- 6 260 966
- US-B1- 6 322 213

## Description

The present invention concerns an eye lens profile capable of correcting ametropias associated with astigmatism.

In general the technical field of the invention is that of contact lenses, but extends also to intra-ocular lenses.

Known to prior art are aspheric optical surfaces defined constructively in such a manner as to enable them to reduce the spherical aberration problem peculiar of a lens constituted by a radius of curvature having its centre on the optical axis of the lens. These constructional definitions generate an undesired linear increase of the dioptric power from the centre to the periphery of the optical zone of such lenses. References to definitions of this kind can be found in Borish - Clinical Refraction, p.1067 (Spherical Aberration) and Anto Rossetti - Manuale di Optometria a Contattologia, p.67 (Ottica Fisiologica).

In the description and the claims that follow use will be made of terms that have the following meanings:
- "internal" and "external", when referred to the surface or the profile of a lens, indicate, respectively, the side or profile of the lens in contact with the eye and the opposite side or surface;
- "progression" is the function according to which the dioptric power of the lens varies with the distance from the centre of the lens to the periphery;
- "basic progression " is the locus of the points that may constitute the progression in accordance with the invention;
- "lens profile" generally - but not exclusively -indicates the external profile of the lens visible in a diametral section of the lens; the profile, in its turn, is representative of the progression. It should be borne in mind that the gradual variation of the progression and the corresponding profile are of the order of hundredths of a millimetre and follow each other about every ten micron as one proceeds from the centre to the periphery of the optical zone, so that the infinite progressions and associated profiles that can characterize the invented lens could be shown in a drawing only by having recourse to a truly enormous scale;
- "visual axis" is the axis that unites the retinal fovea and the observed object;
- the diagrams showing the progression of the lens have the distance in millimetres (mm) of the optical zone from the centre of the lens as abscissa, while the ordinate is constituted by the dioptric power of the lens in dioptres (dio.) - (they are the diagrams of a figure rotating about the geometric axis of the lens).

A common aspheric lens has the purpose not only of compensating a visual defect, but also of improving the quality of the perceived image. The spherical dioptric value of the lens utilized for this purpose corresponds to the dioptric value needed to compensate the visual defect without any de facto improvement of the visual performance at other distances, especially those close to the eye of the user.

From the prior art, Documents EP 0 996 024 A1 and US 6,260,966 B1 are known where the different regions in the lenses appear each dedicated to a specific correction, central vision correction, intermediate vision correction and outer vision correction so that in these regions each of the progression lines is to be considered per se, independently from the others, whilst in the application it would be meaningful considering single progression lines instead of considering the feature characterized by the whole of the progression lines forming each lens, said whole of progression lines being the one that provides the sought effect. Document US 6,322,213 B1, one of the inventors being the inventor in this application, is also known where the scope of the lens is different from the scope of this invention, the first being designed for compensating presbiopia and the second for compensating presbiopia and astigmatism and consequently the two lenses can not be similar nor the first disclosure can be a teaching in this application even if some similarities exist in certain parts of the progression lines. Limit values of the dioptric power are different and the whole of the progression lines is different in the two documents under comparison.

The inventor noted that when one constructs or modifies an aspheric optical surface, no matter whether internal or external, specifically designed for the purpose of increasing the depth of focus (from the point of view of the lens) with consequent increase of the field depth (seen from the side of the person carrying said lens), one obtains multiple positive effects on vision.

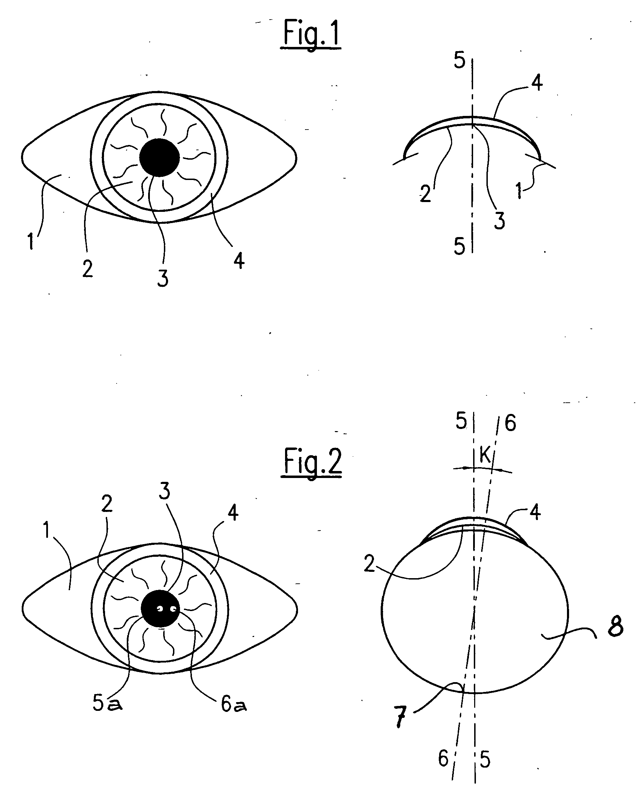

When processing an optical surface having a variable dioptric progression intended for contact with the surface of the cornea, one of the principal obstacles encountered that will generally frustrate - at least partially - the effect of compensating a visual defect is the one due to an erroneous alignment of the optical axis of the contact lens with respect to the visual axis of the human eye. One of the characteristics of the present invention can only be appreciated if one understands what happens when a lens is applied to a corneal surface. The corneal topography, if considered as relating to "spherical" cornea with or without surface astigmatism, is characterized by a curvature greater than that of the surrounding scleral zone (see Figure 1), and this makes it possible for a spherical or toric contact lens to be positioned in accordance with a simple logic that envisages the surface of the lens in question to be constructed with a curvature that reflects (mirrors) the curvature of the cornea beneath it and, facilitated by the viscosity of the lachrymal liquid interposed between them, requires the lens to be "centred" on the cornea surface in order to find the most appropriate geometric position, seeing that the material of which the lens is made, no matter what type it may be, is "elastic" and therefore tends recreate the least possible tension within it.

As can be seen from Figure 1, the geometric axis of the lens coincides with the geometric axis of the cornea; but there exists a complication due to the fact that the "visual axis" does not coincide with this axis (see Figure 2). As noted and stated in literature, de facto there is no coincidence between the antero-posterior geometric axis of the eye (on which there rests also the geometric centre of the surface of the contact lens) and the visual axis. The angle formed between the two axes is known as the angle K; it may vary from one subject to another, generally in the range between 0° and 3.5°, and, bearing in mind that the contact lens is centred on the geometric axis of the eyeball, it therefore follows that any optical effect and power variation impressed on the surface of the contact lens will suffer from this lack of alignment with the visual axis. Indeed, the contact lens, if conventionally constructed, will be characterized by an "axosymmetrical" geometry. In other words, no matter what the desired power variation, it will be generated by starting from the geometric centre of the contact lens, which is circular in shape, and moving towards the periphery of the optical zone. This condition does not imply a deterioration of the function of compensating an optical defect for as long as the power remains homogeneously distributed over the optical surface.

It should here be mentioned that in the subsequent description and in the claims appended hereto the distance in millimetres from the centre of the lens will be indicated more simply with "... mm from the centre" and that the terms "of the order of" and "about", when referred to dioptres are to be understood as meaning that the values stated for the dioptres can vary by:
- ± 0.50 dioptres for values greater than 0 dioptres,
- ± 0.25 dioptres for values lower than 0 dioptres.

The present invention sets out to provide a lens with an optical surface indifferently generated on the inner or outer surface of a corneal contact lens, in a intra-ocular lens. Said lens is to have a profile that confers to the lens a dioptric power having a progression that starts from a maximum value of about 10 dioptres at the centre of the lens, continues to a value of the order of 3.00 dioptres at 0.50 mm from the centre, continues to a value of the order of 0 dioptres at 0.70 mm from the centre, continues further to a value of the order of-0.50 dioptres at 0.88 mm from the centre, thereafter remains substantially parallel to the abscissa until about 1.25 mm from the centre, and then rises again to a value of the order of 1.80 dioptres at 1.32 mm from the centre, then increases even further to about 2.35 dioptres at 1.40 mm from the centre, then re-descends to a value of the order of -0.5 dioptres at 1.40 mm from the centre, continues substantially parallel to the abscissa until about 1.50 mm from the centre, thereafter continues to a value of about 3.00 dpt at 1.50 mm and then continues further to a value of about 3.50 dioptres at 1.60 mm from the centre, then descends again to a value of the order of -0.50 dioptres at 1.70 mm, then descends to a value of about -0.75 dioptres at 2.50 mm from the centre and thereafter the dioptric power remains constant at about -0.75 dioptres until the end of the optical zone of the lens, the value of the dioptres at the centre of the lens varying from a maximum of about 10 dioptres to a minimum of about 1.00 dioptres.

From the point of view of the professional who performs the application (the optometrist), the advantage deriving from the use of a lens made in this manner consists in that he is in a position to "dose" the dioptric power of the lens (increasing it in the positive lenses and reducing it in the negative lenses) with respect to the effective power that would be conventionally assigned to it in accordance with the entity the of the user's visual defect without altering the visual performance of the lens in long-distance viewing.

An example of a real case will be helpful for the purpose of a better understanding of the invention and its advantages.

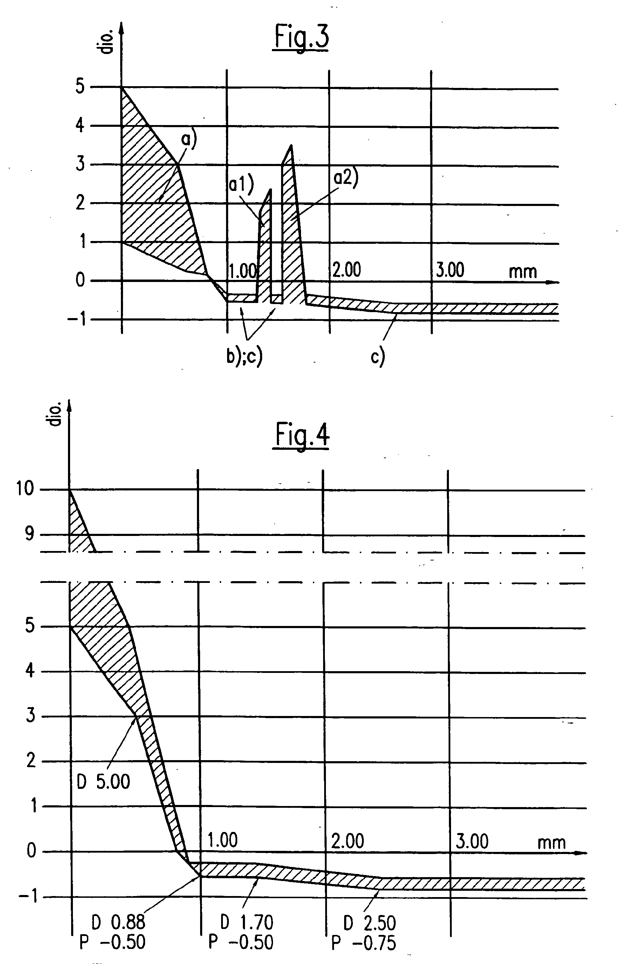

Let us assume an ametropic and far-sighted subject who needs an optical compensation of +2.00 dioptres to attain what is considered a standard 10/10 visus for far vision:
a) if the subject were to be corrected by means of a conventional spherical optical system equal to +2.00 dioptres (a contact lens, for example), he would be in a position to resolve details up to a visus of 10/10, but will not be able to separate or resolve fine details at intermediate distances (for example: =.70 m - 1 metre) or at close distances (conventional reading distance: 30/40 cm).
b) If the same subject were to be corrected with a lens of, for example, +2.50 dioptres (with respect to his ametropia that a correction of +2.00 for far vision) the obtainable visus would amount to 8-9/10; the obvious loss of performance in far vision would however lead to an advantage as far as vision at intermediate distances is concerned, even though it would not yet enable him to read a text at close distance.
c) On the other hand, if the same subject were to be corrected with an optical surface calculated in accordance with the methods proposed by the present invention, i.e. attributing a different dioptric power that varies in a non linear manner from the centre to the periphery of the optical zone in accordance with a progression as illustrated in Figure 3, one notes that, even though the ametropic and far-sighted subject needs an optical compensation of +2.00 dioptres, he is compensated for far vision with - for example - a correction value that ranges from 0.25 to 1.25 dioptres more than the value his ametropia without there being any deterioration of the standard visus for far vision (10/10), accompanied by a considerable and tangible improvement of visual performance at intermediate and reading distances.
d) the same subject, compensated by means of a specific optical surface appropriately calculated by means of the methods proposed by the present invention, i.e. a gain attributing a different dioptric power that varies in a non linear manner from the centre to the periphery of the optical zone in accordance with the progression illustrated in Figure 3 and respecting the nominal value of his ametropia of +2.00, he obtains a visus for far vision equal to about 12-15/10, certainly well above the one of 10/10 hitherto considered standard, but with a smaller visual advantage at intermediate distances than the one described in c) above and insufficient at reading distances.

Everything said above, as also the description that follows, is a consequence of the fact that light is energy and that the visual system captures and encodes the energy comprised in the spectrum of the visible electromagnetic radiations and all the information contained in it; similarly, the optical schemes to which we are so strongly accustomed are no more than schematic reductions of what really happens at the perceptive level. Consequently, it would be wholly useless and opinionable to attribute a specific functioning to each of the different parts of the progression of the invented lens.

Figures 1 and 2, each of which consists of a vertical view and a horizontal section, help to understand the invention; they have been used in the preceding part of the present description and, in particular, are as follows:
Figure 1 shows the sclera 1, the cornea 2, the pupil 3 and the contact lens 4, while the reference number 5 indicates the coincident geometric axes of the eye and the lens;
Figure 2 again shows the parts 1, 2, 3, 4 and 5 indicated in Figure 1, while K is the angle between the axis 5 and the visual axis 6 that unites the retinal fovea 7 and the observed object. In the left-hand part of this figure the reference numbers 5a and 6a indicate, respectively, the points at which the axis 5 and 6 encounter the pupil 3, while the reference number 8 in the right-hand part indicates the eyeball.

A more detailed illustration of the invention will now be provided, complete with an embodiment example, making reference to Figures 3 and 4, two diagrams that relate to the invented lens and have the distance from the centre of the lens as abscissa and the dioptric power as ordinate.

Figure 3 shows the progression of a contact lens that commences from a maximum value at the centre that can vary between about 5.00 dioptres and about 1.00 dioptres and then proceeds as already described in the presentation of the invention, the figure indicating also hatched areas within which the progression may vary; for the hatched areas a1) and a2) this is due to the fact that the progression mat vary around the values defined by the lines of the diagram. Each part or tract comprised in the diagram is to be considered as "something unique" for the purposes of the functioning of the lens, that is to say, each individual part would not be capable of performing any corrective function by itself and ensures that the lens will attain its purpose solely by virtue of the fact that said part is associated with the other parts or tracts of the progression.

Taken together, the areas a), a1) and a2) are dedicated to providing the power needed to provide a clean-cut (sharp) vision at close distances, the area a) is defined by two possible maximum values ranging from 5 to 1.0 dioptres at the centre of the lens and is defined by minimum value at about 0.8 mm from the centre and 0.25 dioptres; a1) and a2) are certainly necessary in lenses for subjects who have an angle K greater than 1° (one degree); the area b) is intended for the pure correction of the subject's ametropia, while the area c) is intended for the correction of a part of the spherical aberration of the eye; the height (thickness) of the areas b) and c) within which the dioptric power may vary amounts to about 0.25 dioptres.

Figure 4 shows the progression of a lens suitable for a subject having an angle K of less than 1° (one degree). This lens no longer needs the areas a1) and a2) and its progression lies within an area defined by an upper line and a lower line as defined hereinbelow (where D indicates the distance in millimetres from the centre of the lens and P indicates the dioptric power of the lens in dioptres):
- the upper line starts from a value of about 10 dioptres at the optical centre of the lens, continues to a value of 5 dioptres at 0.5 mm from the centre, continues to a value of -0.25 dioptres at 0.80 mm from the centre and thereafter continues substantially parallel to the abscissa until 1.70 mm from the centre, subsequently diminishing to -0.50 dioptres at 2.5 mm from the centre and thereafter remaining constant to the end of the optical zone of the lens;
- the lower line starts from a value of about 5 dioptres at the centre of the lens, continues to a value of about 3 dioptres at 0.50 mm, continues to a value of about 0 dioptres at 0.70 mm from the centre, continues to a value of about-0,50 dioptres at 0.88 mm from the centre, continues substantially parallel to the abscissa until 1.70 mm from the centre, subsequently diminishing to a value of about -0.75 dioptres at 2.50 mm and then maintaining the same dioptric power to the end of the optical zone.

The present invention can be applied to any optical surface, irrespective of whether said surface is or is not integral with the surface of the eye, and is particularly indicated for compensating simple ametropias and ametropias associated with astigmatism when one of the surfaces making up the optical system as employed or already set is toric and also for improving vision in the dark.

Summarizing, it should be noted that the invention can be applied to:
- a contact lens, on the anterior or posterior surface;
- an intraocular lens, on the anterior or posterior surface.

## Claims

1. Contact or intraocular eye lens wherein the profile is capable of correcting ametropias associated with astigmatism **characterized in that** it confers to the lens a dioptric power having a progression that starts from a maximum value varying from about 10 dioptres to 1.00 dioptres at the centre of the lens, continues to a value of the order of 3.00 dioptres at 0.50 mm from the centre, continues to a value of the order of 0 dioptres at 0.70 mm from the centre, continues further to a value of the order of -0.50 dioptres at 0.88 mm from the centre, thereafter remains substantially parallel to the abscissa until about 1.25 mm from the centre, and then rises again to a value of the order of 1.80 dioptres at 1.32 mm from the centre, then increases even further to about 2.35 dioptres at 1.40 mm from the centre, then re-descends to a value of the order of -0.5 dioptres at 1.40 mm from the centre, continues substantially parallel to the abscissa until about 1.50 mm from the centre, thereafter continues to a value of about 3.00 dpt at 1.50 mm and then continues further to a value of about 3.50 dioptres at 1.60 mm from the centre, then descends again to a value of the order of -0.50 dioptres at 1.70 mm, then descends to a value of about-0.75 dioptres at 2.50 mm from the centre and thereafter the dioptric power remains constant at about -0.75 dioptres until the end of the optical zone of the lens.

2. Contact or intraocular eye lens wherein the profile is capable of correcting ametropias associated with astigmatism **characterized in that** it confers to the lens a dioptric power whose progression lies within an area defined by an upper line and a lower line as defined hereinbelow
- the upper line starts from a value of about 10 dioptres at the optical centre of the lens, continues to a value of about 5 dioptres at 0.5 mm from the centre, continues to a value of about -0.25 dioptres at 0.80 mm from the centre and thereafter continues substantially parallel to the abscissa until 1.70 mm from the centre, subsequently diminishing to about -0.50 dioptres at 2.50 mm from the centre and thereafter remaining constant to the end of the optical zone of the lens;
- the lower line starts from a value of about 5 dioptres at the centre of the lens, continues to a value of about 3 dioptres at 0.50 mm, continues to a value of about 0 dioptres at 0.70 mm from the centre, continues to a value of about-0,50 at 0.88 mm from the centre, continues substantially parallel to the abscissa until 1.70 mm from the centre, subsequently diminishing to a value of about -0.75 dioptres at 2.50 mm and then maintaining the same dioptric power to the end of the optical zone.

## Patentansprüche

1. Kontakt- oder intraokulare Augenlinse, deren Profil in der Lage ist, mit Astigmatismus verbundene Ametropien zu korrigieren, **dadurch gekennzeichnet, dass** es der Linse eine Brechkraft mit einem Verlauf verleiht, der ausgehend von einem von etwa 10 Dioptrien bis 1,00 Dioptrien variierenden Maximalwert im Zentrum der Linse sich zu einem Wert in der Größenordnung von 3,00 Dioptrien bei 0,50 mm vom Zentrum fortsetzt, sich auf einen Wert in der Größenordung von 0 Dioptrien bei 0,70 mm vom Zentrum fortsetzt, sich weiterhin zu einem Wert in der Größenordung -0,50 Dioptrien bei 0,88 mm vom Zentrum fortsetzt, danach im Wesentlichen parallel zur Abszissenachse bis etwa 1,25 mm vom Zentrum verbleibt, dann erneut auf einen Wert in der Größenordung von 1,80 Dioptrien bei 1,32 mm vom Zentrum ansteigt, danach noch weiter auf etwa 2,35 Dioptrien bei 1,40 mm vom Zentrum zunimmt, dann wieder auf einen Wert in der Größenordnung von - 0,5 Dioptrien bei 1,40 mm vom Zentrum abnimmt, sich im Wesentlichen parallel zur Abszissenachse bis etwa 1,50 mm vom Zentrum fortsetzt, danach zu einem Wert von etwa 3,00 Dioptrien bei 1,50 mm übergeht und dann sich weiter zu einem Wert von etwa 3,50 Dioptrien bei 1,60 mm vom Zentrum fortsetzt, dann erneut auf einen Wert in der Größenordung von -0,50 Dioptrien bei 1,70 mm absinkt, dann auf einen Wert von etwa -0,75 Dioptrien bei 2,50 mm vom Zentrum abnimmt, und danach die Brechkraft bei etwa -0,75 Dioptrien bis zum Ende der optischen Zone der Linse konstant bleibt.

2. Kontakt- oder intraokulare Augenlinse, deren Profil in der Lage ist, mit Astigmatismus verbundene Ametropien zu korrigieren, **dadurch gekennzeichnet, dass** es der Linse eine Brechkraft verleiht, deren Verlauf in einem Bereich liegt, der von einer oberen und einer unteren Linie begrenzt ist, die wie folgt definiert sind:
- die obere Linie geht aus von einem Wert von etwa 10 Dioptrien im optischen Zentrum der Linse, setzt sich fort zu einem Wert von etwa 5 Dioptrien bei 0,5 mm vom Zentrum, setzt sich fort zu einem Wert von etwa -0,25 Dioptrien bei 0,80 mm vom Zentrum und verläuft danach im Wesentlichen parallel zu der Abszissenachse bis 1,70 mm vom Zentrum, nimmt anschließend auf etwa -0,50 Dioptrien bei 2,50 mm vom Zentrum ab und bleibt danach konstant bis zum Ende der optischen Zone der Linse;
- die untere Linie geht aus von einem Wert von etwa 5 Dioptrien im Zentrum der Linse, setzt sich fort zu einem Wert von etwa 3 Dioptrien bei 0,50 mm, setzt sich fort zu einem Wert von etwa 0 Dioptrien bei 0,70 mm vom Zentrum, setzt sich fort zu einem Wert von 0,50 bei 0,88 mm vom Zentrum, verläuft weiter im Wesentlichen parallel zu der Abszissenachse bis 1,70 mm vom Zentrum, um anschließend abzunehmen auf einen Wert von -0,75 Dioptrien bei 2,50 mm und anschließend dieselbe Brechkraft bis zum Ende der optischen Zone beizubehalten.

## Revendications

1. Lentille de contact oculaire ou intraoculaire, dont le profil est capable de corriger des amétropies associées à l'astigmatisme,
**caractérisée en ce que**
le profil confère à la lentille une puissance dioptrique présentant une progression qui part d'une valeur maximum variant d'environ 10 dioptries jusqu'à 1,00 dioptrie au centre de la lentille, se poursuit jusqu'à une valeur de 3,00 dioptries à 0,50 mm du centre, se poursuit jusqu'à une valeur de l'ordre de 0 dioptrie à 0,70 mm du centre, se poursuit encore jusqu'à une valeur de l'ordre de -0,50 dioptrie à 0,88 mm du centre, reste ensuite essentiellement parallèle à l'abscisse jusqu'à environ 1,25 mm du centre, puis augmente de nouveau jusqu'à une valeur de l'ordre de 1,80 dioptrie à 1,32 mm du centre, augmente ensuite encore plus jusqu'à environ 2,35 dioptrie à 1,40 mm du centre, redescend ensuite jusqu'à 1,40 mm du centre, se poursuit essentiellement parallèlement à l'abscisse jusqu'à environ 1,50 mm du centre, se poursuit ensuite jusqu'à une valeur d'environ 3,00 dioptries à 1,50 mm puis se poursuit encore jusqu'à une valeur d'environ 3,50 dioptries à 1,60 mm du centre, redescend ensuite jusqu'à une valeur de l'ordre de -0,50 dioptrie à 1,70 mm, descend ensuite jusqu'à une valeur d'environ -0,75 dioptrie à 2,50 mm du centre, et la puissance dioptrique reste ensuite constante à environ -0,75 dioptrie jusqu'à la fin de la zone optique de la lentille.

2. Lentille de contact oculaire ou intraoculaire, dont le profil est capable de corriger des amétropies associées à l'astigmatisme,
**caractérisée en ce que**
le profil confère à la lentille une puissance dioptrique dont la progression se situe à l'intérieur d'une zone définie par une ligne supérieure et une ligne inférieure telles que définies ci-après :
- la ligne supérieure part d'une valeur d'environ 10 dioptries au centre optique de la lentille, se poursuit jusqu'à une valeur d'environ 5 dioptries à 0,50 mm du centre puis se poursuit jusqu'à une valeur d'environ -0,25 dioptrie à 0,80 mm du centre, et se poursuit ensuite essentiellement parallèlement à l'abscisse jusqu'à 1,70 mm du centre, puis diminue jusqu'à environ -0,50 dioptrie à 2,50 mm du centre et reste ensuite constante jusqu'à la fin de la zone optique de la lentille,
- la ligne inférieure part d'une valeur d'environ 5 dioptries au centre de la lentille, se poursuit jusqu'à une valeur d'environ 3 dioptries à 0,50 mm du centre, se poursuit jusqu'à une valeur d'environ 0 dioptrie à 0,70 mm du centre, se poursuit jusqu'à une valeur d'environ -0,50 dioptrie à 0,88 mm du centre, se poursuit essentiellement parallèlement à l'abscisse jusqu'à 1,70 mm du centre, et diminue ensuite jusqu'à une valeur d'environ -0,75 dioptrie à 2,50 mm, puis conserve enfin la même puissance dioptrique jusqu'à la fin de la zone optique.
